# EUROPEAN PATENT APPLICATION

(11) **EP 4 691 546 A1**
(43) Date of publication of application: **11.02.2026**
(21) Application number: 24793034.0
(22) Date of filing: 18.04.2024
(51) Int. Cl.: A61N 1/32, A61N 1/06, A61N 1/04, A61N 1/36, A61B 18/14, A61N 1/40, A61B 18/00

(54) **HIGH FREQUENCY OUTPUT DEVICE**

(30) Priority: 18.04.2023 KR 20230050934; 17.04.2024 KR 20240051514
(71) Applicant: Jeisys Medical Inc., Seoul 08501 (KR)
(72) Inventor: PARK, Ji Hoon, Seoul 08501 (KR)
(74) Representative: dompatent
(86) International application number: PCT/KR2024/005222
(87) International publication number: WO 2024/219849

(57) **Abstract**

A high-frequency output device including includes: a handpiece; a base detachably coupled to the handpiece; a first electrode provided on the base and including at least one first unit electrode; a second electrode provided on the base and including at least one second unit electrode; and a power source configured to supply power to the first electrode and the second electrode.

## Description

### [TECHNICAL FIELD]

The present disclosure relates to a high-frequency output device.

### [BACKGROUND ART]

Skin care devices that treat skin by removing wrinkles, restoring skin elasticity, and removing sebum are being developed. This is because cleanly managed skin makes people look younger and contributes to an attractive appearance.

Types of skin care devices include a type of delivering ultrasound to the skin (HIFU type), a type of delivering high-frequency waves to the skin (RF type), and a type of delivering laser light to the skin (Optical type).

A high-frequency output device that delivers high-frequency waves to the skin applies high-frequency waves generated from the RF electrode to the skin through the epidermis of the skin. As a result, as the high-frequency waves are delivered to the skin, coagulation necrosis of the skin is induced, so that collagen and elastic fibers of the skin are removed and new collagen and elastic fibers can be formed. In addition, coagulation necrosis of the skin is also effective in improving skin pigmentation, acne scars, and wrinkles.

The high-frequency output device can be classified into a bipolar type including an RF electrode of the first polarity and an RF electrode of the second polarity, and a monopolar type in which the RF electrode has a single polarity and a ground electrode is provided separately.

In the bipolar type, the current applied to the RF electrode of the first polarity flows to the RF electrode of the second polarity, and the high-frequency waves are oscillated in a relatively narrow area between the RF electrode of the first polarity and the RF electrode of the second polarity, so that the high-frequency waves can be intensively applied to the skin.

In the monopolar type, the current applied to a plurality of RF electrodes of the same polarity flows to a counter electrode plate coupled to another part of the skin (e.g., the abdomen and back of the body), and the high-frequency waves are oscillated in a relatively wide area between the plurality of RF electrodes and the counter electrode plate, so that the high-frequency waves can be applied not only to the skin but also to the surrounding area of the skin

For the monopolar type, when a plurality of RF electrodes is arranged in a cross-like manner and a unidirectional current is applied to the plurality of RF electrodes, the current may be offset due to the magnetic field generated in the plurality of RF electrodes, and the current applied to the plurality of RF electrodes may flow in a biased manner toward the edges or centers of the plurality of RF electrodes, thereby being biasedly oscillated.

In this way, the phenomenon in which the high-frequency waves oscillated from the plurality of RF electrodes flow in a biased manner toward the edges of the crossed electrodes among the plurality of RF electrodes or flow in a biased manner toward the center of the crossed electrodes among the plurality of RF electrodes can be called the proximity effect.

However, when the proximity effect occurs in the monopolar type, there is a problem that the skin improvement effect is reduced because the high-frequency waves oscillated from the plurality of RF electrodes are biasedly distributed only to a specific area of the skin.

### [DETAILED DESCRIPTION OF THE INVENTION]

### [TECHINICAL PROBLEM]

The present disclosure provides a high-frequency output device capable of preventing the occurrence of the proximity effect when a monopolar type high-frequency output is performed.

In addition, the present disclosure provides a high-frequency output device including electrodes arranged in a pattern shape capable of outputting at least one of monopolar type high-frequency waves or bipolar type high-frequency waves.

Technical problems of the inventive concept are not limited to the technical problems mentioned above, and other technical problems not mentioned will be clearly understood by those skilled in the art from the following description.

### [TECHNICAL SOLUTION]

In an aspect of the present disclosure, a high-frequency output device includes a handpiece; a base detachably coupled to the handpiece; a first electrode provided on the base and including at least one first unit electrode; a second electrode provided on the base and including at least one second unit electrode; and a power source configured to supply power to the first electrode and the second electrode.

Furthermore, the first unit electrode and the second unit electrode may be alternately arranged on the base.

Furthermore, the second unit electrode may be interposed between two neighboring first unit electrodes.

Furthermore, the first electrode may include a first contact terminal connecting the first unit electrode and the power source, and the second electrode may include a second contact terminal connecting the second unit electrode and the power source.

Furthermore, the first unit electrode may have a shape extending from the first contact terminal toward the second contact terminal, and the second unit electrode may have a shape extending from the second contact terminal toward the first contact terminal.

Furthermore, a single first unit electrode may be arranged at a center of the base, a plurality of the second unit electrodes may be arranged at an edge of the base, and at least two of the second unit electrodes among the plurality of the second unit electrodes may be arranged to face each other.

Furthermore, a plurality of the first unit electrodes may be arranged at a center of the base, a plurality of the second unit electrodes may be arranged at an edge of the base, and two of the second unit electrodes among the plurality of the second unit electrodes may be arranged to face each other.

Furthermore, the power source may be configured to apply currents of different polarities to two of the second unit electrodes that are arranged to face each other among the plurality of the second unit electrodes, respectively.

Furthermore, the first unit electrode and the second unit electrode may be formed as at least one of a non-invasive type, a film type, or an invasive type, respectively.

Furthermore, the non-invasive type may include a polygonal shape or a disc shape, and the invasive type may include a needle shape.

Furthermore, the device may further include a processor configured to control the power source so that a high-frequency pattern including at least one of a monopolar type high-frequency and a bipolar type high-frequency or output from the first unit electrode and the second unit electrode.

Furthermore, the high-frequency pattern may include: a first single pattern in which only the monopolar type high-frequency is output; a second single pattern in which only the bipolar type high-frequency is output; a first alternating pattern in which the monopolar type high-frequency and the bipolar type high-frequency are output alternately; a second alternating pattern in which the bipolar type high-frequency and the monopolar type high-frequency are output alternately; and a simultaneous pattern in which the monopolar type high-frequency and the bipolar type high-frequency are output simultaneously.

Furthermore, the power source may be configured to apply two currents having an identical polarity in different directions to the first unit electrode and the second unit electrode.

Other specific details of the present disclosure are included in the detailed description and drawings.

### [ADVANTAGEOUS EFFECTS OF THE INVENTION]

According to the present disclosure, the high-frequency output device according to one embodiment of the present disclosure has an effect of preventing the occurrence of the proximity effect when outputting a monopolar type high-frequency.

In addition, the high-frequency output device according to one embodiment of the present disclosure has an effect of being able to output at least one of a monopolar type high-frequency and a bipolar type high-frequency.

The effects of the present disclosure are not limited to the effects mentioned above, and other effects not mentioned will be clearly understood by those skilled in the art from the description.

### [DESCRIPTION OF THE DRAWINGS]

FIG. 1A is a perspective view illustrating a high-frequency output device according to an embodiment of the present disclosure.
FIG. 1B is a block diagram illustrating a high-frequency output device according to an embodiment of the present disclosure.
FIG. 2 is a schematic diagram illustrating a state in which a first electrode and a second electrode of a high-frequency output device according to an embodiment of the present disclosure output a monopolar type high-frequency.
FIG. 3 is a schematic diagram illustrating a state in which a first electrode and a second electrode of a high-frequency output device according to an embodiment of the present disclosure output a bipolar type high-frequency.
FIGS. 4A to 4C are schematic diagrams illustrating a state in which a first electrode and a second electrode of a high-frequency output device according to an embodiment of the present disclosure output a bipolar type high-frequency. FIGS. 5 to 12 are schematic diagrams illustrating various embodiments of a high-frequency output device according to another embodiment of the present disclosure.

### [BEST MODE]

The advantages and features of the present disclosure, and methods for achieving them, will become clear with reference to the embodiments described in detail below along with the accompanying drawings. However, the present disclosure is not limited to the embodiments disclosed below and may be implemented in various different forms. The present embodiments are merely provided to ensure that the present disclosure is complete, and provided to fully convey the scope of the present disclosure to those skilled in the art to which the present disclosure pertains.

The terminology used herein is for the purpose of describing embodiments and is not intended to limit the disclosure. As used herein, singular forms also include plural forms, unless specifically stated otherwise in the context. As used in the specification, "comprises" and/or "comprising" does not exclude the presence or addition of one or more other elements in addition to the mentioned elements. Throughout the specification, the same reference numerals refer to the same elements, and "and/or" includes each and every combination of the elements mentioned. Although "first", "second", etc. are used to describe various elements, it is to be understood that these elements are not limited by these terms. These terms are merely used to distinguish one element from another. Accordingly, it should be understood that a first element mentioned below may also be a second element within the technical scope of the present invention.

Unless otherwise defined, all terms (including technical and scientific terms) used in this specification may be used with meanings commonly understood by those skilled in the art to which this disclosure pertains.

Hereinafter, embodiments of the present disclosure will be described in detail with reference to the attached drawings.

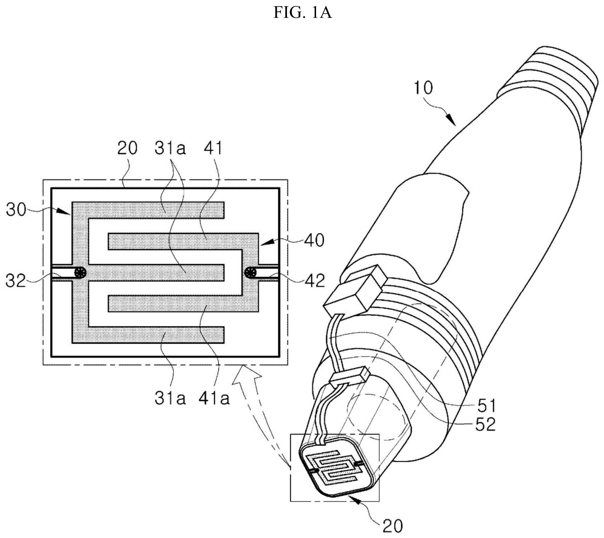

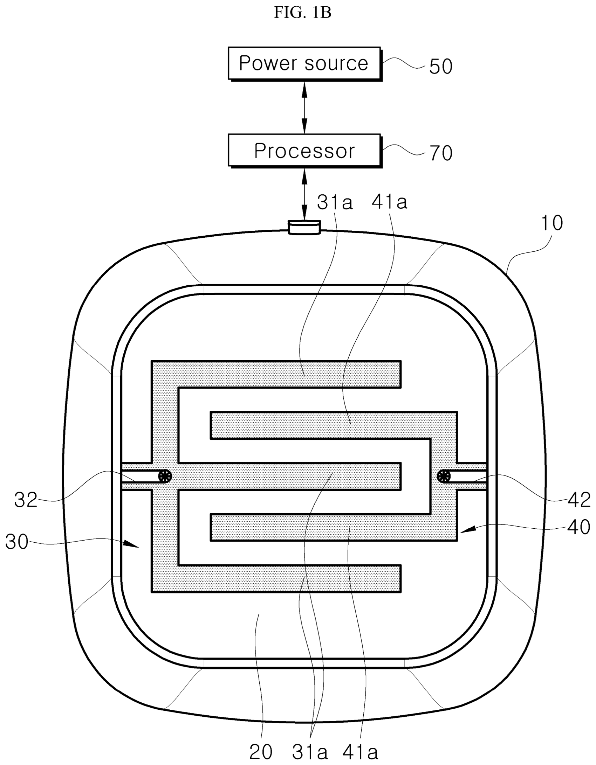

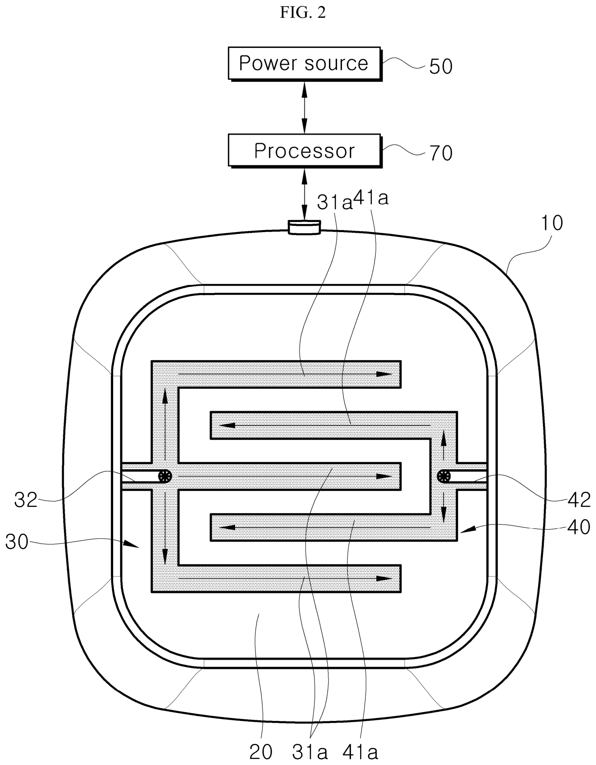

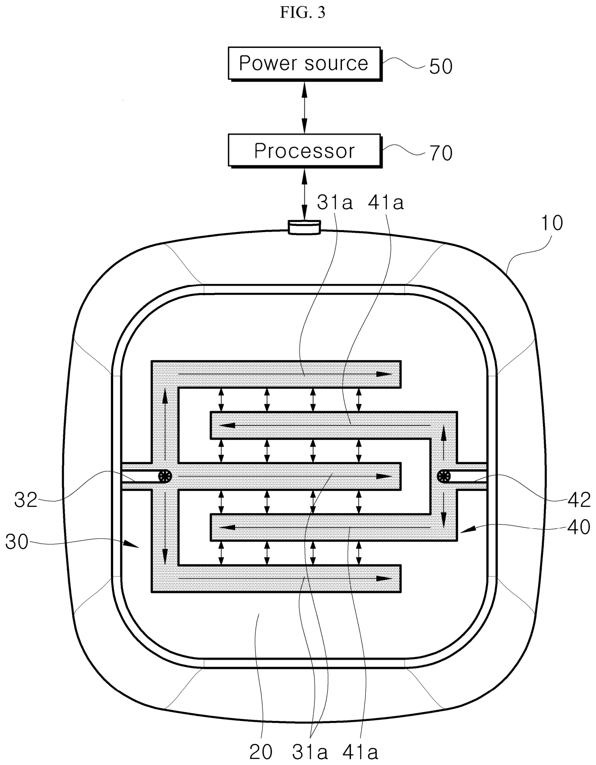

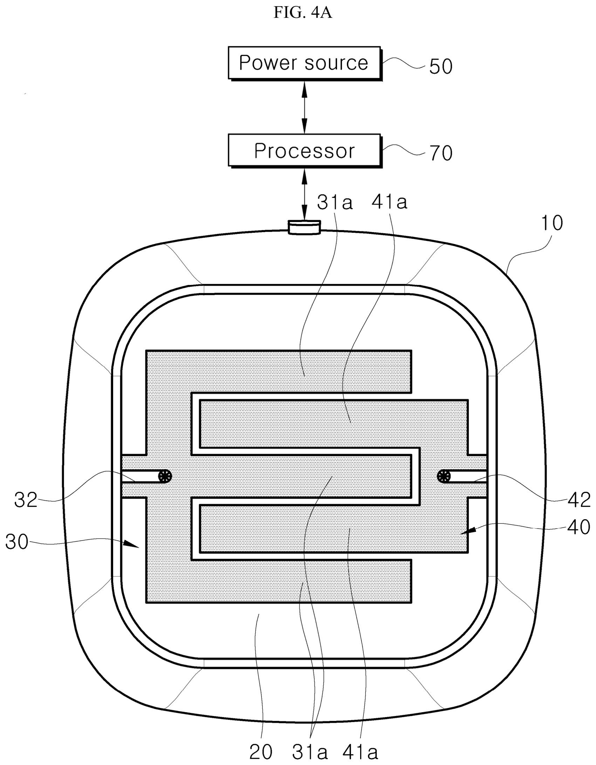

FIG. 1A is a perspective view illustrating a high-frequency output device according to an embodiment of the present disclosure, FIG. 1B is a block diagram illustrating a high-frequency output device according to an embodiment of the present disclosure, FIG. 2 is a schematic diagram illustrating a state in which a first electrode and a second electrode of a high-frequency output device according to an embodiment of the present disclosure output a monopolar type high-frequency, FIG. 3 is a schematic diagram illustrating a state in which a first electrode and a second electrode of a high-frequency output device according to an embodiment of the present disclosure output a bipolar type high-frequency, and FIGS. 4A to 4C are schematic diagrams illustrating a state in which a first electrode and a second electrode of a high-frequency output device according to an embodiment of the present disclosure output a bipolar type high-frequency.

As shown in FIGS. 1A and 1B, a high-frequency output device according to an embodiment of the present disclosure may include a high-frequency output device main body, a handpiece 10, a base 20, a first electrode 30, a second electrode 40, a power source 50, and a processor 70.

The high-frequency output device main body serves as a basic body of the present disclosure. The high-frequency output device main body may be detachably coupled to the handpiece 10 and may be connected by wire or wirelessly. Here, the handpiece 10 may be detachably coupled to the base 20. For example, the handpiece 10 and the base 20 may be detachably coupled through a hook groove and a fixing hook. Here, the handpiece 10 may have a hook groove formed, and the base 20 may have a fixing hook detachably fixed to the hook groove formed. In addition, the handpiece 10 may have a fixing hook formed, and the base 20 may have a hook groove to which the fixing hook is detachably fixed.

The power source 50, a first cable 51, and a second cable 52 may be provided inside the high-frequency output device main body.

The power source 50 serves to apply current to the first electrode 30 and the second electrode 40. The first cable 51 may connect the power source 50 and the first electrode 30. The second cable 52 may connect the second electrode 40.

For example, a power button for turning the power source 50 on/off and two control buttons for adjusting the intensity of the current applied from the power source 50 to the first electrode 30 and the second electrode 40 may be provided on the outer surface of the main body of the high-frequency output device. When the power source 50 is turned on by the user's operation, current may be applied from the power source 50 to at least one of the first electrode 30 or the second electrode 40.

The base 20 may serve to fix the first electrode 30 and the second electrode 40. The base 20 may be detachably coupled to one side of the handpiece 10. For example, the base 20 may have a plate shape. In addition, the base 20 may be provided with the first electrode 30 and the second electrode 40.

The first electrode 30 may be provided on the base 20 and may include at least one first unit electrode 31a. The second electrode 40 may be provided on the base 20 and may include at least one second unit electrode 32.

Referring to FIG. 1B, a first unit electrode 31a and a second unit electrode 41a may be alternately arranged on the base 20. Here, one end of the first unit electrode 31a may be arranged to be misaligned with the neighboring second unit electrode 41a, and one end of the second unit electrode 41a may be arranged to be misaligned with the neighboring first unit electrode 31a. In addition, the second unit electrode 41a may be interposed between two neighboring first unit electrodes 31a. In this way, as the first unit electrodes 31a and the second unit electrodes 41a are arranged alternately, when currents of the same polarity are applied from the power source 50 to the first unit electrode 31a and the second unit electrode 41a, the monopolar type high-frequency output from the first unit electrode 31a and the second unit electrode 41a is not deflected toward the center of the base 20. Accordingly, the proximity effect in which the monopolar type high-frequency output from the first unit electrode 31a and the second unit electrode 41a is deflected toward the center or the outside of the first unit electrode 31a and the second unit electrode 41a may be prevented.

Meanwhile, the power source 50 may prevent the proximity effect in which the monopolar type high-frequency output from the first unit electrode 31a and the second unit electrode 41a is deflected toward the center or the outside of the first unit electrode 31a and the second unit electrode 41a by applying two currents having the same polarity to the first unit electrode 31a and the second unit electrode 41a in different directions under the control of the processor 70.

The first electrode 30 may include a first contact terminal 32 connecting the first unit electrode 31a and the power source 50.

The second electrode 40 may include a second contact terminal 42 connecting the second unit electrode 41a and the power source 50.

For example, the first contact terminal 32 and the second contact terminal 42 may be printed circuit boards (PCBs).

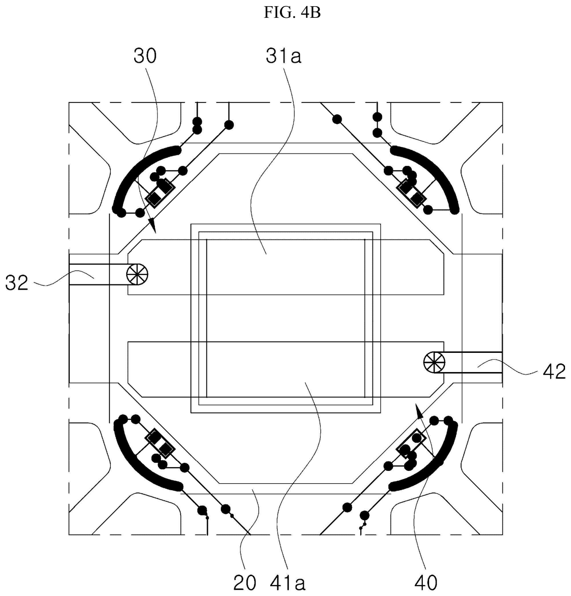

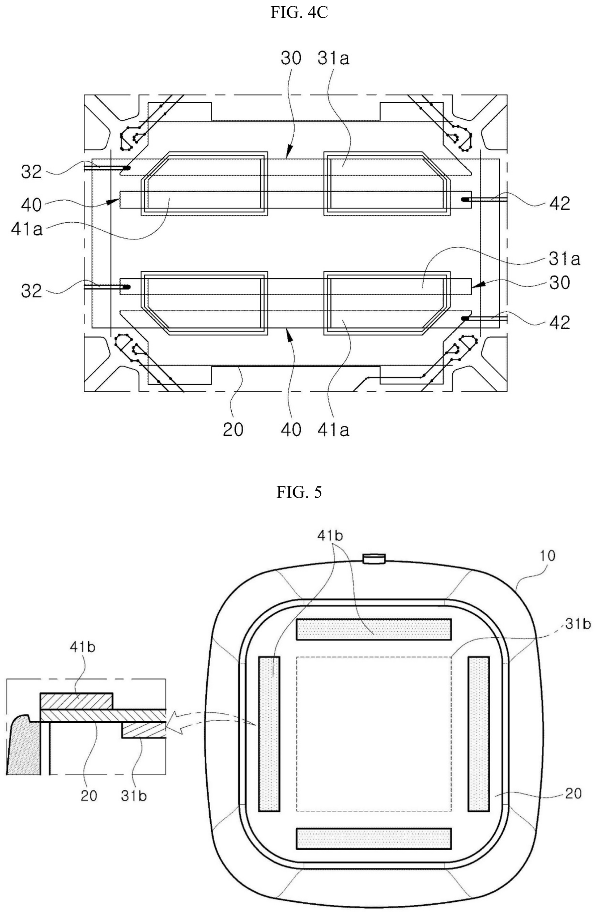

For example, the first unit electrode 31a may have a shape that extends vertically from the first contact terminal 32 toward the second contact terminal 42. In addition, the second unit electrode 41a may have a shape that extends vertically from the second contact terminal 42 toward the second contact terminal 32 (See FIGS. 4B and 4C).

The present disclosure may further include a switching circuit. Here, the first contact terminal 32 may connect the switching circuit to the first unit electrode. In addition, the second contact terminal 42 may connect the switching circuit to the second unit electrode. The power source 50 may apply current of the same polarity to the plurality of first unit electrodes 31a and the plurality of second unit electrodes 41a through the switching circuit. In addition, the power source 50 may apply current of the same polarity to at least one of the plurality of first unit electrodes 31a and at least one of the plurality of second unit electrodes 41a through the switching circuit. The power source 50 may apply current of the same polarity to at least one of the plurality of first unit electrodes 31a or at least one of the plurality of second unit electrodes 41a through the switching circuit. In addition, the power source 50 may apply current of different polarities to two second unit electrodes 41a that are arranged opposite to each other among the plurality of second unit electrodes 41a through the switching circuit under the control of the processor 70.

The processor 70 may control the power source 50 so that a high-frequency pattern including at least one of a monopolar type high-frequency or a bipolar type high-frequency is output from the first unit electrode 31a and the second unit electrode 41a. Here, the high-frequency pattern may be transmitted to the skin.

The high-frequency pattern may include a first single pattern in which only a monopolar type high-frequency is output, a second single pattern in which only a bipolar type high-frequency is output, a first alternating pattern in which a monopolar type high-frequency and a bipolar type high-frequency are output alternately, and a second alternating pattern in which a bipolar type high-frequency and a monopolar type high-frequency are output alternately.

For example, since two currents having the same polarity but different directions are applied from the power source 50 to the first unit electrode 31a and the second unit electrode 41a under the control of the processor 70, only a high-frequency of the monopolar type may be output to the skin from the first unit electrode 31a and the second unit electrode 41a (See FIG. 3). Specifically, a current of the first polarity and the first direction is applied from the power source 50 to the first unit electrode 31a, a current of the second polarity and the first direction different from the first polarity and the first direction is applied to the second unit electrode 41a, and a current of the second polarity may be applied from the power source 50 to a counter electrode plate placed on another part of the skin. Accordingly, the high-frequency of the monopolar type outputted to the skin from the first unit electrode 31a and the second unit electrode 41a may flow to the counter electrode plate. As illustrated in FIG. 2, when the power source 50 applies the same polarity current of the monopolar type in different directions to the first unit electrode 30 and the second unit electrode 40, the proximity effect may be prevented as the same polarity current of the monopolar type is applied in different directions to the first unit electrode 31a and the second unit electrode 41a. As another example, the first single pattern may output only a monopolar type high-frequency to the skin from the first unit electrode 30 or the second unit electrode 40 as the same polarity current is applied from the power source 50 to the first unit electrode 31a or the second unit electrode 41a under the control of the processor 70.

As an example, the second single pattern may output only a bipolar type high-frequency to the skin from the first unit electrode 31a and the second unit electrode 41a as two currents of different polarities are applied from the power source 50 to the first unit electrode 31a and the second unit electrode 41a under the control of the processor 70 (See FIG. 3). At this time, the first unit electrode 31a and the second unit electrode 41a may be in contact with the skin. In addition, the bipolar type high-frequency output from the first unit electrode 31a and the second unit electrode 41a may be circulated between the first unit electrode 31a and the second unit electrode 41a.

For example, the first alternating pattern may alternately repeat the first single pattern and the second single pattern under the control of the processor 70.

For example, the second alternating pattern may alternately repeat the second single pattern and the first single pattern under the control of the processor 70.

The processor 70 may include a user interface that selectively receives the first single pattern, the second single pattern, the first alternating pattern, and the second alternating pattern. Here, the processor 70 may control the power source 50 according to the control signal input to the user interface. For example, the user interface may use a touch pad, a keyboard, a button, and the like. For example, the processor may be a microcomputer or a PLC (Programmable Logic Controller).

For example, compared to the first unit electrode 31a and the second unit electrode 41a of FIGS. 1A to 3, the first unit electrode 31a and the second unit electrode 41a of FIG. 4A may have a thicker thickness. Accordingly, as the range of high-frequency output from the first unit electrode 31a and the second unit electrode 41a is widened, the high-frequency may be applied to a wider area of the skin.

For example, as shown in FIG. 4B, the first contact terminal 32 and the second contact terminal 42 may each be configured as a single unit. Here, a single first contact terminal 32 may connect the power supply 50 to a single first unit electrode 31, and a single second contact terminal 42 may connect the power supply 50 to a single second unit electrode 41, respectively.

As an example, as shown in FIG. 4C, the first contact terminal 32 and the second contact terminal 42 are constructed in plural, and the plurality of first contact terminals 32 may connect the power supply 50 to the plurality of first unit electrodes 31a, respectively, and the plurality of second contact terminals 42 may connect the power supply 50 to the plurality of second unit electrodes 41a, respectively. However, in FIG. 4C, an example is shown in which there are two of the first contact terminals 32 and two of the second contact terminals 42, and two of the first unit electrodes 31a and two of the second unit electrodes 41a, but the present disclosure is not limited thereto, and there may be two or more of the first contact terminals 32 and two or more of the second contact terminals 42, and there may also be two or more of the first unit electrodes 31a and two or more of the second unit electrodes 41a.

In this embodiment, the plurality of first contact terminals 32 may connect the power supply 50 to the plurality of first unit electrodes 31, respectively, and the plurality of second contact terminals 42 may connect the power supply 50 to the plurality of second unit electrodes 41, respectively, in parallel.

Hereinafter, various embodiments of a high-frequency output device according to another embodiment of the present disclosure will be described.

FIGS. 5 to 12 are schematic diagrams illustrating various embodiments of a high-frequency output device according to another embodiment of the present disclosure.

As shown in FIGS. 5 to 12, the first unit electrode and the second unit electrode may be formed as at least one of a non-invasive type, a film type, or an invasive type, respectively.

The non-invasive type may include a polygonal shape, a disc shape, and a plate shape.

For example, the first unit electrode of the non-invasive type may have any one of a polygonal shape, a disc shape, and a plate shape. However, the present disclosure is not limited thereto, and may have various other shapes.

In addition, the second unit electrode of the non-invasive type may have any one of a polygonal shape, a disc shape, and a plate shape. However, the present disclosure is not limited thereto, and may have various other shapes.

The invasive type may include a needle shape. For example, the first unit electrode of the invasive type may have a needle shape. In addition, the second unit electrode of the invasive type may have a needle shape.

The film type may be an electrode film surrounding the base 20 or the base 20 itself may be an electrode film, and the first unit electrode and the second unit electrode may be arranged on this electrode film. For example, the first unit electrode of the film type may be located on the inner surface of the electrode film. Therefore, when viewed from the outside of the handpiece 10, the first unit electrode of the film type may have a form covered by the electrode film. In addition, the second unit electrode of the film type may be arranged on the outer surface of the electrode film.

The power source 50 may apply a current of the same polarity to at least one of the plurality of first unit electrodes through the first cable 51 under the control of the switching circuit of the processor 70, and at the same time, may apply a current of the same polarity as the current applied to the first unit electrode to at least one of the plurality of second unit electrodes through the second cable 52. In addition, the power source 50 may apply currents of different polarities to two second unit electrodes that are arranged opposite each other among the plurality of second unit electrodes under the control of the processor 70 of the processor 70.

As an example, the power source 50 may apply two currents having the same polarity in different directions to the first unit electrode and the second unit electrode that are arranged opposite each other under the control of the switching circuit of the processor 70.

In one embodiment, referring to FIGS. 5 to 8, a single first unit electrode 31b or 31c is arranged at the center of the base 20, a plurality of second unit electrodes 41b and 41c are arranged at the edge of the base 20, and at least two second unit electrodes 41b and 41c among the plurality of second unit electrodes 41b and 41c may be arranged to face each other.

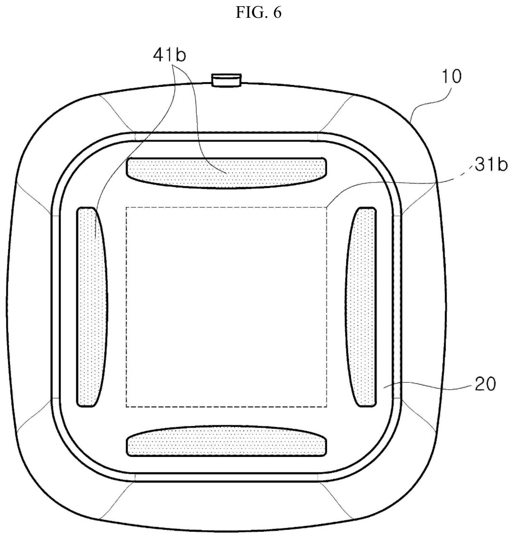

Referring to FIGS. 5 and 6, the single first unit electrode 31b is arranged at the center of the base 20, the two second unit electrodes 41b among the plurality of second unit electrodes 41b are arranged to face each other in a first direction at the edge of the base 20, and the two second unit electrodes 41b among the plurality of second unit electrodes 41b may be arranged to face each other in a second direction at the edge of the base 20. At this time, the first direction may be the X-axis direction or the left-right direction, and the second direction may be the Y-axis direction or the up-down direction. Here, the first unit electrode 31b and the second unit electrode 41b may be formed as a non-invasive type. Specifically, referring to FIG. 5, the first unit electrode 31b and the second unit electrode 41b may have a plate shape. Also, referring to FIG. 6, the first unit electrode 31b may have a plate shape, the second unit electrode 41b may have a disk shape, and the curved surface of the second unit electrode 41b may be arranged to face the first unit electrode 31b.

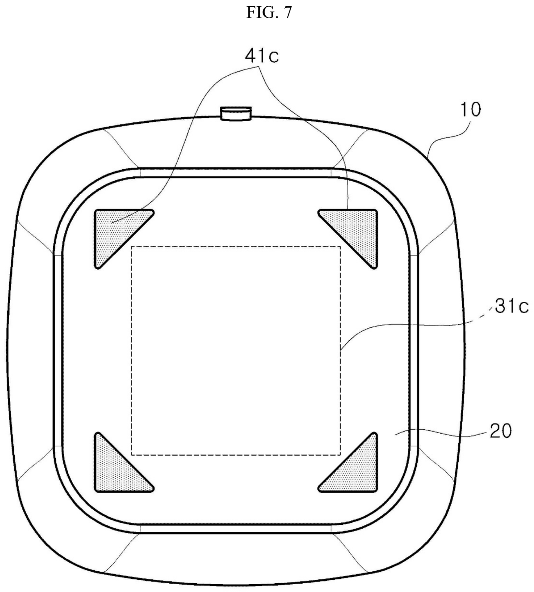

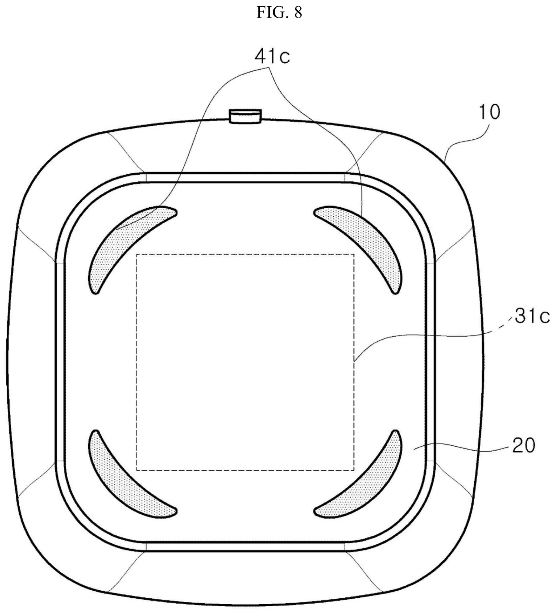

Referring to FIGS. 7 and 8, the single first unit electrode 31c may be arranged at the center of the base 20, and the plurality of second unit electrodes 41c may be arranged at each corner of the base 20. Specifically, the four second unit electrodes 41c may be arranged at each of the first corner, the second corner, the third corner, and the fourth corner of the base 20. Here, the first corner may be located at the upper left side of the base 20, the second corner may be located at the upper right side of the base 20, the third corner may be located at the lower left side of the base 20, and the fourth corner may be located at the lower right side of the base 20. Here, the first unit electrode 31c and the second unit electrode 41c may be formed as a non-invasive type. Specifically, referring to FIG. 7, the first unit electrode 31c may have a plate shape, and the second unit electrode 41c may have a triangular shape. In addition, referring to FIG. 8, the first unit electrode 31c may have a plate shape, and the second unit electrode 41c may have an arc shape.

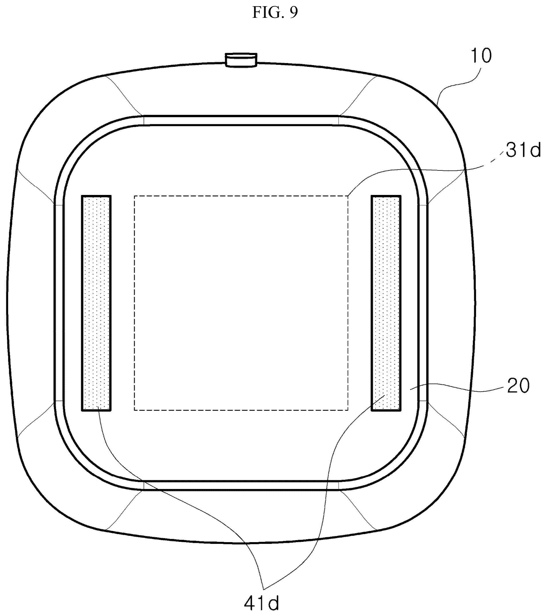

Referring to FIG. 9, a single first unit electrode 31d is arranged at the center of the base 20, and two second unit electrodes 41d may be arranged to face each other in the first direction or the second direction on the edge of the base 20. Here, the first unit electrode 31d and the second unit electrode 41d may be formed as a non-invasive type. Specifically, referring to FIG. 9, the first unit electrode 31d and the second unit electrode 41d may have a plate shape.

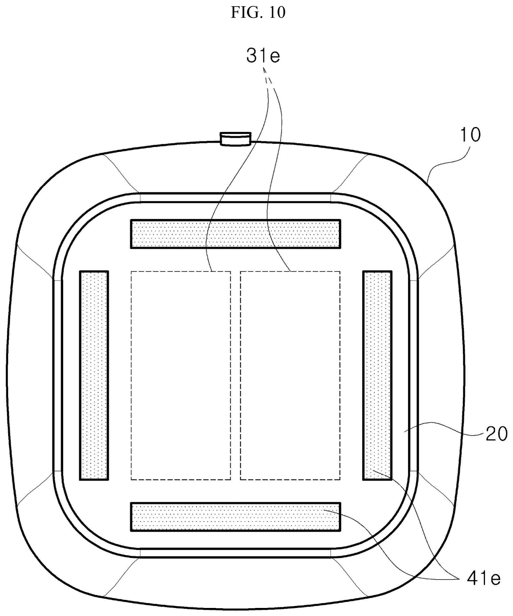

In one embodiment, referring to FIGS. 10 to 12, a plurality of first unit electrodes 31e are arranged at the center of the base 20, a plurality of second unit electrodes 41e are arranged at the edge of the base 20, and two of the second unit electrodes 41e among the plurality of second unit electrodes 41e may be arranged to face each other.

Referring to FIG. 10, the plurality of first unit electrodes 31e are arranged in a second direction at the center of the base 20, and the plurality of second unit electrodes 41e may be arranged as in the embodiments of FIGS. 6 and 7. Here, the first unit electrodes 31e and the second unit electrodes 41e may be formed as a non-invasive type. Specifically, referring to FIG. 10, the first unit electrodes 31e and the second unit electrodes 41e may have a plate shape.

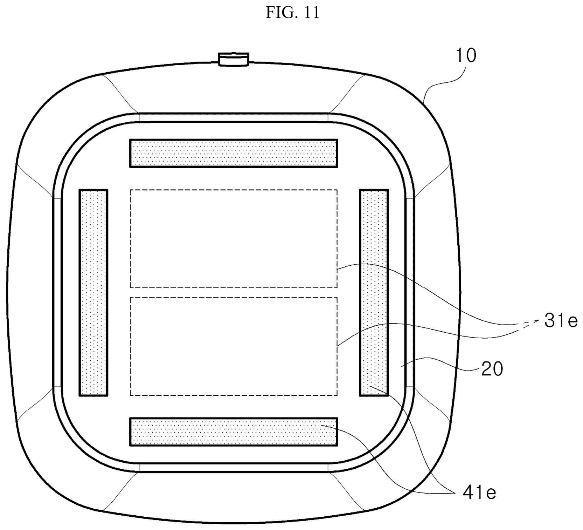

Referring to FIG. 11, the plurality of first unit electrodes 31e are arranged in a first direction at the center of the base 20, and the plurality of second unit electrodes 41e may be arranged as in the embodiments of FIGS. 6 and 7. Here, the first unit electrodes 31e and the second unit electrodes 41e may be formed as a non-invasive type. Specifically, referring to FIG. 11, the first unit electrode 31e and the second unit electrode 41e may have a plate shape.

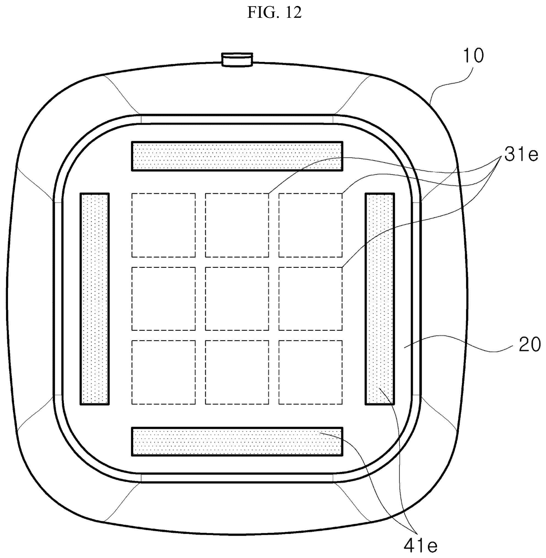

Referring to FIG. 12, the plurality of first unit electrodes 31e are arranged in a grid shape at the center of the base 20, and the plurality of second unit electrodes 41e may be arranged as in the embodiments of FIGS. 6 and 7. Here, the first unit electrode 31e and the second unit electrode 41e may be formed in a non-invasive type. Specifically, referring to FIG. 12, the first unit electrode 31e and the second unit electrode 41e may have a plate shape.

In the present embodiment, the processor 70 may control the power source 50 so that a high-frequency pattern including at least one of a monopolar type high-frequency or a bipolar type high-frequency is output from the first unit electrodes 31b to 31e and the second unit electrodes 41b to 41e. Here, the high-frequency pattern may be transmitted to the skin.

In the present embodiment, the high-frequency pattern may include the first single pattern in which only a monopolar type high-frequency is output, the second single pattern in which only a bipolar type high-frequency is output, the first alternating pattern in which a monopolar type high-frequency and a bipolar type high-frequency are output alternately, the second alternating pattern in which a bipolar type high-frequency and a monopolar type high-frequency are output alternately, and the simultaneous pattern in which a bipolar type high-frequency and a monopolar type high-frequency are output simultaneously.

For example, the first single pattern may output only a high-frequency of a monopolar type to the skin from the first unit electrode and the second unit electrode by applying two currents with the same polarity but different directions from the power source 50 to the first unit electrode and the second unit electrode under the control of the processor 70. In addition, a current of the first polarity and the first direction is applied to the first unit electrode from the power source 50, a current of the second polarity and the first direction different from the first polarity and the first direction is applied to the second unit electrode from the power source 50, and a current of the second polarity may be applied to the counter electrode plate placed on another part of the skin from the power source 50. Therefore, the high-frequency of a monopolar type output to the skin from the first unit electrode and the second unit electrode may flow to the counter electrode plate.

As another example, the first single pattern may output only a monopolar type high-frequency to the skin from the first unit electrode or the second unit electrode as the current of the same polarity is applied to the first unit electrode or the second unit electrode from the power source 50 under the control of the processor 70.

The second single pattern may output only a bipolar type high-frequency to the skin from the two second unit electrodes that are arranged opposite each other as the two currents of different polarities are applied to the two second unit electrodes that are arranged opposite each other from the power source 50 under the control of the processor 70. At this time, the two second unit electrodes that are arranged opposite each other may contact or invade the skin. In addition, the bipolar type high-frequency output from the two second unit electrodes that are arranged opposite each other may be circulated between the two second unit electrodes that are arranged opposite each other.

The first alternating pattern may alternately repeat the first single pattern and the second single pattern under the control of the processor 70.

The second alternating pattern may alternately repeat the second single pattern and the first single pattern under the control of the processor 70.

The simultaneous pattern may alternately repeat the second single pattern and the first single pattern under the control of the processor 70. As a current of the same polarity is applied from the power source 50 to the first unit electrode and two currents of different polarities are applied to the two second unit electrodes that are arranged opposite each other, a monopolar type high-frequency may be output to the skin from the first unit electrode, and at the same time, a bipolar type high-frequency may be output to the skin from the two second unit electrodes that are arranged opposite each other.

In the present embodiment, the processor 70 may include the user interface that selectively receives the first single pattern, the second single pattern, the first alternating pattern, the second alternating pattern, and the simultaneous pattern. Here, the processor 70 may control the power source 50 according to a control signal input to the user interface. For example, the user interface may use a touch pad, a keyboard, a button, and the like.

According to the present disclosure, the high-frequency output device according to one embodiment of the present disclosure has an effect of preventing the occurrence of the proximity effect when outputting a monopolar type high-frequency.

In addition, the high-frequency output device according to one embodiment of the present disclosure has an effect of being able to output at least one of a monopolar type high-frequency or a bipolar type high-frequency.

Although the embodiments of the present disclosure have been described above with reference to the attached drawings, those skilled in the art will understand that the present disclosure may be implemented in other specific forms without changing the technical idea or essential features thereof. Therefore, it should be understood that the embodiments described above are exemplary in all respects and not restrictive.

## Claims

1. A high-frequency output device, comprising:
a handpiece;
a base detachably coupled to the handpiece;
a first electrode provided on the base and including at least one first unit electrode;
a second electrode provided on the base and including at least one second unit electrode; and
a power source configured to supply power to the first electrode and the second electrode.

2. The device according to claim 1,
wherein the at least one first unit electrode and the at least one second unit electrode are alternately arranged on the base.

3. The device according to claim 2,
wherein the at least one second unit electrode is interposed between two neighboring first unit electrodes.

4. The device according to claim 2,
wherein the first electrode includes a first contact terminal connecting the at least one first unit electrode and the power source, and
wherein the second electrode includes a second contact terminal connecting the at least one second unit electrode and the power source.

5. The device according to claim 4,
wherein the at least one first unit electrode has a shape extending from the first contact terminal toward the second contact terminal, and
wherein the at least one second unit electrode has a shape extending from the second contact terminal toward the first contact terminal.

6. The device according to claim 1,
wherein a single first unit electrode is arranged at a center of the base,
wherein a plurality of the second unit electrodes is arranged at an edge of the base, and
wherein at least two of the second unit electrodes among the plurality of the second unit electrodes are arranged to face each other.

7. The device according to claim 1,
wherein a plurality of the first unit electrodes is arranged at a center of the base,
wherein a plurality of the second unit electrodes is arranged at an edge of the base, and
wherein two of the second unit electrodes among the plurality of the second unit electrodes are arranged to face each other.

8. The device according to claim 7,
wherein the power source is configured to respectively apply currents of different polarities to the two second unit electrodes that are arranged to face each other.

9. The device according to claim 1,
wherein the at least one first unit electrode and the at least one second unit electrode are respectively formed as at least one of a non-invasive type, a film type, or an invasive type.

10. The device according to claim 1, wherein the at least one first unit electrode and/or the at least one second unit electrode are respectively formed as a non-invasive type, wherein the non-invasive type includes a polygonal shape or a disc shape.

11. The device according to claim 1, further comprising:
a processor configured to control the power source so that a high-frequency pattern including at least one of a monopolar type high-frequency or a bipolar type high-frequency is output from the at least one first unit electrode and the at least one second unit electrode.

12. The device according to claim 9, wherein the high-frequency pattern includes:
a first single pattern in which only the monopolar type high-frequency is output;
a second single pattern in which only the bipolar type high-frequency is output;
a first alternating pattern in which the monopolar type high-frequency and the bipolar type high-frequency are output alternately;
a second alternating pattern in which the bipolar type high-frequency and the monopolar type high-frequency are output alternately; and
a simultaneous pattern in which the monopolar type high-frequency and the bipolar type high-frequency are output simultaneously.

13. The device according to claim 12,
wherein the power source is configured to apply two currents having an identical polarity in different directions to the at least one first unit electrode and the at least one second unit electrode.
